# EUROPEAN PATENT APPLICATION

(11) **EP 2 990 042 A1**
(43) Date of publication of application: **02.03.2016**
(21) Application number: 14787556.1
(22) Date of filing: 18.04.2014
(51) Int. Cl.: A61K 31/7048, A61K 9/14, A61K 9/20, A61K 31/702, A61K 36/00, A61K 36/18, A61K 36/28, A61K 36/899, A61K 47/26, A61P 1/00, A61P 1/02, A61P 1/04, A61P 1/10, A61P 1/14, A61P 1/16, A61P 3/00, A61P 3/02, A61P 3/04, A61P 3/06

(54) **COMPOSITION COMPRISING FRUCTOOLIGOSACCHARIDE AND QUERCETIN GLYCOSIDE**

(30) Priority: 26.04.2013 JP 2013093490
(71) Applicant: Suntory Holdings Limited, Kita-ku, Osaka-shi Osaka 530-8203 (JP)
(72) Inventor: SATOU, Yousuke, Soraku-gun Kyoto 619-0284 (JP); TSUJIMOTO, Aki, Soraku-gun Kyoto 619-0284 (JP)
(74) Representative: Vossius & Partner Patentanwälte Rechtsanwälte mbB
(86) International application number: PCT/JP2014/061045
(87) International publication number: WO 2014/175180

(57) **Abstract**

A quercetin glycoside and a fructooligosaccharide are mixed at a weight ratio of the fructooligosaccharide to the quercetin glycoside being 0.1 or greater to thereby suppress the bitterness of the quercetin glycoside in a quercetin glycoside-containing oral composition.

## Description

### TECHNICAL FIELD

The present invention relates to an oral composition comprising a fructooligosaccharide and a quercetin glycoside.

### BACKGROUND ART

As public interest in health has grown, attention has been given to an attempt to maintain good health through daily consumption of foods. In particular, there has been a dramatic progress in the development of oral compositions comprising a biofunctional component, and those compositions have been provided in various forms of products.

Examples of such a biofunctional component include those components of plant origin. For example, glycosides of quercetin, a type of flavonoids which is rich in plants like onion and buckwheat, have been known to exhibit various physiological actions. Hitherto, quercetin glycosides have been reported to have antiinflammatory, antioxidative, vasoconstrictive, capillary wall-strengthening and other activities, and have been incorporated typically in foods and pharmaceutical and cosmetic products. Also, it has been reported in recent years that quercetin glycosides are capable of suppressing protein degradation (Non-patent Literature 1). However, quercetin glycosides have peculiar bitterness which becomes problematic when they are provided in the form of foods and beverages, etc. As a method for masking such bitterness, there has been known a bitterness masking method using a chondroitin sulfate-containing cartilage extract with an aqueous solvent (Patent Literature 1).

### CITATION LIST

### PATENT LITERATURE

Patent Literature 1: Japanese Patent No. JP 4652486

### NON-PATENT LITERATURE

Non-patent Literature 1: Molecular and Cellular Biochemistry 243: 153-160, 2003

### SUMMARY OF INVENTION

### TECHNICAL PROBLEM

As mentioned above, components having peculiar bitterness, like quercetin glycosides, often become problematic in terms of their flavor and palatability when they are orally consumed. In a particular case, where bitterness components are intended to be orally consumed on a daily basis in the form of functional foods such as supplements, it is preferred to suppress the bitterness of those bitterness components and thereby improve their palatability. The present invention has as its objects to provide a new method for suppressing the bitterness of an oral composition comprising a functional component having bitterness, like a quercetin glycoside, as well as to provide a new oral composition having suppressed bitterness which is obtained by said method.

### SOLUTION TO PROBLEM

The present inventors have made intensive studies on a bitterness suppression method and, as a result, have found that bitterness can be masked by adding a fructooligosaccharide to a bitterness component (quercetin glycoside) at a particular ratio; thus, the inventors have completed the present invention.

### ADVANTAGEOUS EFFECTS OF INVENTION

According to the present invention, the bitterness of a quercetin glycoside can be effectively suppressed by incorporating a fructooligosaccharide in a quercetin glycoside-containing oral composition at a particular ratio. Also, the composition of this invention, which has suppressed bitterness, is characterized by being highly palatable and easy to consume on a continuous basis.

Further, the oral composition of the present invention is provided not only with the health promoting function of a quercetin glycoside but also with the intestinal regulation activity of a fructooligosaccharide. In addition to intestinal regulation activity, fructooligosaccharides are known to have a mineral absorption-promoting effect, a caries-retarding effect, a feces odor-improving effect, and other effects, so the inventive oral composition is expected to produce such effects.

Furthermore, fructooligosaccharides are capable of serving as a binding agent, as described below; thus, the oral composition of the present invention can be granulated into granules or tabletted into tablets, even without the use of a widely used binding agent like hydroxypropylcellulose. In general, consumers have a tendency not to like compositions abundant in additives other than biofunctional components for reasons of health and palatability, and given this fact, the oral composition which is characterized by containing little or no widely used binding agent like hydroxypropylcellulose is advantageous in that it is easily accepted by consumers.

Since the fructooligosaccharide in the inventive oral composition serves as a binding agent, the oral composition is also advantageous in that it can be effectively shaped into granules even when, for example, an insoluble, poorly adhesive, powdery ingredient containing dietary fiber is added thereto. Dietary fiber-containing ingredients are known to have various efficacies; thus, the composition comprising not only a biofunctional component like a quercetin glycoside, and a fructooligosaccharide, but also a dietary fiber-containing ingredient is preferable from health and functional viewpoints. Further, a granule, or a tablet formed with the granule, is a preferred form from the viewpoint of ease of consumption. Furthermore, since the fructooligosaccharide is capable of masking bitterness but does not have much influence on the flavor of dietary fiber-containing ingredients (e.g., vegetables), this is advantageous in that it can provide a highly palatable product that allows one to enjoy the inherent flavor of dietary fiber-containing ingredients like vegetables.

### DESCRIPTION OF EMBODIMENTS

The present invention relates to an oral composition comprising a quercetin glycoside and a fructooligosaccharide at a particular ratio. This invention also relates to a method for suppressing the bitterness of a quercetin glycoside by using a fructooligosaccharide.

### <Quercetin glycoside>

The quercetin glycoside refers to a glycoside of quercetin, a type of flavonoids, and can be exemplified by rutin, quercitrin, isoquercitrin and the like, as well as enzymatically treated products thereof. Quercetin glycosides have been known to have various physiological actions, and have been reported to produce antiinflammatory, antioxidative, vasoconstrictive, capillary permeability-reducing, and capillary wall-strengthening activities as well as to be capable of inhibiting some enzymes involved in cell growth and the like.

The quercetin glycoside to be used in the inventive composition is not particularly limited as to its origin or preparation method. For example, known examples of plants from which a quercetin glycoside can be obtained include caper, apple, tea plant, onion, grape, broccoli, *Corchorus olitorius,* raspberry, lingonberry, cranberry, Opuntia, leaf vegetables, and citrus fruits.

The quercetin glycoside to be used in the inventive composition is preferably rutin or an analog thereof. Rutin, a type of flavonoids, is known as a vitamin-like substance since it acts like a vitamin. Rutin can be obtained typically from plants of the family *Rutaceae,* and also from sophora belonging to the family *Liguminosae* and buckwheat belonging to the family *Polygonaceae.*

In a preferred mode, there can be used a product obtained by subjecting a quercetin glycoside to a sugar transfer reaction through treatment with a glycosyltransferase or the like. Quercetin glycosides like rutin are, in some cases, difficult to use because they are hardly soluble in water, whereas enzymatically treated products of quercetin glycosides are improved in terms of their water solubility by a sugar transfer reaction and thus can be used advantageously in the composition of the present invention.

In a particularly preferred mode, an enzymatically treated product of rutin (hereinafter referred to as "enzymatically treated rutin") can be used as a quercetin glycoside. The enzymatically treated rutin refers to a product obtained by enzymatically treating rutin or an analog thereof. The analog of ruin can be exemplified by quercitrin and isoquercitrin. Rutin is generally known to have antioxidative activity but has limited use because of being hardly soluble in water; however, the water solubility of run can be improved by subjecting it to a sugar transfer reaction to make it into enzymatically treated rutin. Enzymatically treated rutin is known to have not only strong antioxidative activity but also diverse physiological functions such as platelet aggregation/adhesion-inhibiting activity, vasodilative activity, and anticancer activity, and are suitable for use in health foods intended to produce various effects like improvement in inflammation and promotion of blood circulation. Enzymatically treated rutin can be obtained by, for example, subjecting an extract from sophora, buckwheat, or the like to treatment with a glycosyltransferase, as per a method typically disclosed in Japanese Patent Application Publication Nos. JP H07-10898 and JP 2003-33164. In the process of formulation, not only enzymatically treated rutin but also any pharmaceutically acceptable additive may be added.

The amount of the quercetin glycoside to be incorporated in the composition of the present invention is desired to be determined so that the daily consumption of the quercetin glycoside per individual be in the range of 1 to 500 mg, preferably 5 to 500 mg, more preferably 5 to 300 mg, yet more preferably 10 to 300 mg. Alternatively, the daily consumption of the quercetin glycoside per kg of body weight can be adjusted to be in the range of, for example, 0.02 to 10 mg/kg, preferably 0.10 to 6.0 mg/kg. Further, the proportion of the quercetin glycoside to be incorporated in the inventive composition can be adjusted to be in the range of 0.1 to 95% by weight, preferably 0.3 to 80% by weight, more preferably 0.3 to 10% by weight, based on the total of the composition.

### <Fructooligosaccharide>

In the composition of the present invention, a fructooligosaccharide is incorporated at a particular ratio with respect to a bitterness component. Components having bitterness, such as a quercetin glycoside, are unfit for consumption as foods on a daily basis, but mixing the fructooligosaccharide with the quercetin glycoside at a particular ratio suppresses bitterness, thereby producing an easy-to-consume oral composition.

The fructooligosaccharide is a generic name for mixtures having a structure in which 1 to 3 fructose molecules are linked to the fructose residue of sucrose, and those mixtures are called 1-kestose, nystose, and 1F-β-fructofuranosylnystose in ascending order of chain length. Fructooligosaccharides are contained in vegetables and fruits such as onion, shallot, garlic, and banana. Fructooligosaccharides can also be produced by enzymatically treating the source material sucrose typically obtained from sugar cane.

Fructooligosaccharides are widely known to have intestinal regulation activity including bifidobacterium growth activity, and are also known to have a mineral absorption-promoting effect, a caries-retarding effect, a feces odor-improving effect, and other effects.

The relative content of the fructooligosaccharide in the oral composition of the present invention is adjusted to be 0.1 or greater, preferably 5 or greater, more preferably 10 or greater (all in weight ratio), with respect to the quercetin glycoside which is taken as 1. Adding the fructooligosaccharide at such a relative ratio suppresses the inherent bitterness of the quercetin glycoside. The upper limit for the content of the fructooligosaccharide is not particularly limited. However, even if the fructooligosaccharide is added in an amount greater than a certain amount, the effect of the fructooligosaccharide will no longer be further enhanced; thus, it may well be desirable that the relative content of the fructooligosaccharide be not greater than 50 (in weight ratio) with respect to the quercetin glycoside which is taken as 1. The relative content of the fructooligosaccharide with respect to the total solids is preferably in the range of 3 to 20% by weight.

### <Oral composition>

The oral composition of the present invention can be used as foods, pharmaceutical products, food additives, and the like. In the inventive composition, the quercetin glycoside having bitterness is contained but the bitterness is suppressed; thus, the composition can be consumed on a daily basis without strain and is suitable for use as foods like so-called functional foods.

As described above, the oral composition of the present invention comprises the quercetin glycoside and the fructooligosaccharide at a particular relative ratio. The composition may also contain other various health components or various additives which are acceptable as foods or pharmaceutical drugs. The inventive composition can be of any form suitable for oral administration, such as tablet, capsule, granule, powder, or lozenge.

The fructooligosaccharide contained in the composition of the present invention can function as a binding agent for binding powdery materials. Thus, the inventive oral composition may be shaped (granulated) into granules by using the fructooligosaccharide as a binding agent. A granule is advantageous not only in that it is harder to scatter and more satisfactory in flowability than a powder, but also in that it is easy to handle when dividedly packed in a bag as an article of manufacture or when removed from such a bag by a consumer. Preferred examples of such granules are granules obtained by granulation so that the proportion of fine powder of 75 µm or less is not greater than a certain value, for example, not greater than 50% by weight. Whether granules obtained by granulation have such a particle size range can be verified by measuring the particle size distribution of the granules. The thus-obtained granules may be further shaped into tablets using a tabletting machine.

The method for granulating the oral composition of the present invention into granules is not particularly limited, and the granulation can be done by any method such as fluidized bed granulation or extruding granulation. In particular, there can advantageously be used fluidized bed granulation which can produce a granulated product with good dispersibility. Addition of the fructooligosaccharide may be achieved by mixing it with a powdery ingredient before a granulation process or by adding, spaying or pouring a solution of the fructooligosaccharide in water onto a powdery ingredient during a granulation process. For example, the granulation may be done by first mixing the fructooligosaccharide with other material and then spraying water onto the mixture, or by spaying a solution of the fructooligosaccharide in water onto a powdery ingredient during a granulation process.

In the usual case of granulating a powdery oral ingredient, a binding agent such as hydroxypropylcellulose, hydroxypropylmethylcellulose, methylcellulose, carboxymethylcellulose, Pullulan, starch, dextrin, guar gum, or galactomannan is used. However, since the fructooligosaccharide acts as a binding agent in the present invention, a granular form of the composition can be produced with the aforementioned binding agent other than the fructooligosaccharide only in a small amount or without the binding agent. The resulting granular form of the composition is satisfactory in dispersibility in water, less prone to scatter as fine powder, and easy to pack dividedly as an article of manufacture. In general, for reasons of health and palatability, consumers do not like products abundant in additives other than biofunctional components -- for example, such other additives include the aforementioned binding agent like hydroxypropylcellulose. Since the inventive composition can be granulated using the fructooligosaccharide which is a biofunctional component, a widely used binding agent like hydroxypropylcellulose may be present only in a small amount or need not be present at all, so the composition may well be desirable to consumers.

The binding agent other than the fructooligosaccharide can be used in such an amount that the solubility and dispersibility of the resulting granular form of the composition in water are not impaired. Though such an amount depends on the strength of the binding agent to be used, the binding agent other than the fructooligosaccharide can be used, for example, in an amount of not greater than 3% by weight when used as a solution, or in an amount of not greater than 10% by weight when applied as a powder.

In the oral composition of the present invention, a powdery ingredient can be granulated by the fructooligosaccharide; thus, even when a water-insoluble, poorly-adhesive ingredient such as a dietary fiber-containing ingredient is applied, granulation can be achieved satisfactorily. Dietary fiber has various effects, mainly including increase in faecal weight, influence on digestive organs, and influence on the metabolism of nutritional components. Among such physiological activities, many studies have been made in the fields of protein metabolism, fat metabolism, and sugar metabolism. Also, studies on the physiological effects against a group of bacteria living in the intestines have received attention. Dietary fiber is mainly rich in fiber-containing parts of plants, such as leaf, bud, stem, blossom, nut, root, spike, seed, and fruit. Dietary fiber-containing ingredients are typically ingredients containing green plant fiber. Preferred examples include food ingredients for use in producing so-called green juice, such as *Ashitaba (Angelica keiskei),* young cereal leaf, green tea plant, kale, young sweet potato leaf, broccoli, *Corchorus olitorius,* young sesame leaf, mulberry leaf, *Peucedanum japonicum,* mugwort and mixtures thereof. *Ashitaba* is preferred in that it is abundant in antioxidative vitamins. Other examples of a dietary fiber-containing ingredient that can be used are disclosed in many publications such as Japanese Patent Application Publication Nos. JP 2003-334046 and JP 2003-79339. The dietary fiber-containing ingredient may be used if necessary after subjected to pretreatment like cutting or blanching as disclosed in Japanese Patent Application Publication No. JP 2002-218945. The dietary fiber-containing ingredient is not particularly limited as to its form and preparation method, but is preferably of a powdery form, more preferably of the form of a powder of 75 µm or less.

*"Ashitaba* (*Angelica keiskei*)" is a perennial herb of the family *Apiaceae.* In Japan, it is cultivated mainly in the Kyushu district as well as in some Kanto district areas adjacent to the Pacific Ocean. *Ashitaba* is abundant in coumarins, chalcones, and other antioxidative vitamins, and is efficacious against arteriosclerosis, constipation and anemia. The plant site to be used is not particularly limited, and all sites including leaf, bud, stem, and fruit can be used. A method for treating *Ashitaba* when used as a dietary fiber-containing ingredient is typically disclosed in Japanese Patent Application Publication Nos. JP S59-154935 and JP H02-231057, but is not particularly limited to those methods disclosed therein in the present invention.

"Young cereal leaf" refers to young leaves of cereal crops, and is specifically exemplified by young leaves of barley, wheat, rye, oats, adlay, and the like. Young cereal leaf is abundant in vitamins, minerals, dietary fiber, and the like, and attracts attention as a health food ingredient having various effects including adsorption of toxic substances, improvement in intestinal environment, inhibition of cholesterol absorption, prevention of a rapid elevation of postparandial blood glucose level, and activation of superoxide dismutase (SOD). A method for treating young cereal leaf when used as a dietary fiber-containing ingredient is typically disclosed in Japanese Patent Application Publication Nos. JP H07-241176, JP 2001-112435, JP 2002-58449, JP 2002-212, JP 2003-9812, and JP 2003-178, but is not particularly limited to those methods disclosed therein in the present invention.

"Green tea plant" refers to non-fermented tea leaves whose fermentation is stopped immediately after being picked up. There are various known preparation methods for green tea leaves, and any preparation method can be used in the present invention. For example, *Ara-cha* (crude tea), *Kona-cha* (dust tea), *Matcha* (powdered green tea), *Sencha* (brewed green tea), *Bancha* (coarse tea) and *Gyokuro* (refined green tea) made by common preparation methods, as well as pulverized products thereof, can be used alone or in admixture thereof (in any blend thereof). A *Sencha* powder and *Matcha,* as well as a blend thereof, are particularly preferred from the viewpoints of savor and cost.

"Kale" is a perennial herb plant belonging to the genus *Brassica* under the family *Brassicaceae,* and was originally an improved variety of cabbage. This plant, whose leaves are abundant in vitamins, is effective in prevention of gastritis and gastric ulcers, and improvement in liver functions and constipation. Kale can be used without particular limitation as long as it comes under "kale of the family *Brassicaceae."* For example, there can be used various varieties of kale, including Siberian Kale, Scotch Kale, and collard. A method for treating kale when used as a dietary fiber-containing ingredient is typically disclosed in Japanese Patent Application Publication Nos. JP 2002-186445, JP 2002-125612, JP 2002-119245, JP 2002-119239, JP 2002-112701, and JP 2002-85010, but is not particularly limited to those methods disclosed therein in the present invention.

"Young sweet potato leaf" refers to stems and leaves of sweet potato belonging to the family *Convolvulaceae.* It is known to have inhibitory effects on increase in postparandial blood glucose level and blood pressure, and on accumulation of liver fat.

"Broccoli" refers to stems and leaves of broccoli of the family *Brassicaceae.* It is abundant in vitamin B, vitamin C, carotene, iron, etc., and is expected to be effective in improvement in blood circulation, etc.

*"Corchorus olitorius"* refers to young leaves of *Corchorus olitorius* (*moroheiya*) belonging to the family *Tiliaceae Juss.* It is abundant in nutrients such as vitamins, carotene, potassium, calcium, and iron.

"Young sesame leaf" refers to young leaves of the plants belonging to the genus *Sesamun* under the family *Pedaliaceae.* It is abundant in nutrients such as polyphenol, folic acid, and iron.

"Mulberry leaf" refers to leaves of the plants belonging to the genus *Morus* under the family *Moraceae.* It is known to have hypotensive, hypoglycemic, analgesic, antimicrobial, expectorant, diuretic, and perspiratory effects.

*"Peucedanum japonicum"* refers to stems and leaves of *Peucedanum japonicum,* a plant of the family *Apiaceae* which is also called *"sakuna".* It is abundant in vitamins, carotene, and calcium.

"Mugwort" refers to stems and leaves of mugwort belonging to the family *Asteraceae.* It has a strengthening effect on the stomach as well as other beneficial effects on abdominal pain, diarrhea, anemia, cold constitution, etc.

The oral composition comprising not only the biofunctional component like the quercetin glycoside, and the fructooligosaccharide, but also the above-described dietary fiber-containing ingredient, is preferable since it can be expected to exhibit not only the biological functions of the quercetin glycoside and the fructooligosaccharide, but also the effects of the dietary fiber-containing ingredient. Further, since the fructooligosaccharide is capable of masking the bitterness of the quercetin glycoside but does not have much influence on the savor of the dietary fiber-containing ingredient like *Ashitaba* or green tea, the present invention makes it possible to prepare an oral composition having reduced bitterness and increased palatability while keeping the inherent savor of such a dietary ingredient.

In addition to the aforementioned components, the oral composition of the present invention may also contain any additive or auxiliary component commonly used in foods or pharmaceutical drugs. For example, an excipient such as starch, lactose, crystalline cellulose, maltitol, dextrin, Pullulan and guar gum, a sweetening agent such as aspartame, xylitol, sucralose, mannitol and galactose, a toughening agent such as carotene, L-ascorbic acid, α-tocopherol, lutein and lycopene, and/or other components may be included.

The oral composition of the present invention may be provided along with a labeling of its functions. A method for the labeling of functions is not particularly limited, and can be exemplified by labeling on a package of a food product, on the surface of a container, in instructions for a food product, in an advertising leaflet for a food product, and the like. Examples of the functions of the inventive oral composition include: prevention or amelioration of symptoms such as arteriosclerosis, anemia, gastritis, gastric ulcer, and constipation; improvement in liver functions; inhibition of toxic substances adsorption; improvement in intestinal environment; dieting effect; inhibition of cholesterol absorption; prevention of a rapid elevation of postparandial blood glucose level; activation of superoxide dismutase; intestinal regulation activity including bifidobacterium growth activity; and colorectal cancer-preventing, mineral absorption-promoting, or antioxidative activity.

Hereunder, the present invention will be described by way of working examples, but these examples are not intended to limit the invention.

### EXAMPLES

### Example 1

Investigation was made to find an oligosaccharide that is capable of advantageously masking the bitterness of a quercetin glycoside in a quercetin glycoside-containing composition. To be specific, a fructooligosaccharide, a maltooligosaccharide, paratinose, raffinose, lactulose, a xylooligosaccharide, and an agarooligosaccharide were tested for their masking effect on the bitterness of the quercetin glycoside.

### <Materials>

The materials used are as follows.
Quercetin glycoside: SAN EMIQ® P15 (produced by San-Ei Gen F. F. I., Inc.; containing 15% quercetin glycoside)
Fructooligosaccharide: Meioligo® P (Powder) (produced by Meiji Food Materia Co., Ltd.; containing 95% fructooligosaccharide)
Maltooligosaccharide: Pine Oligo 20 (produced by Matsutani Chemical Industry Co., Ltd.; containing 65% maltooligosaccharide)
Paratinose: Powder Paratinose ICP (produced by Mitsui Sugar Co., Ltd.; containing 99% paratinose)
Raffinose: Nitten Raffinose® (produced by Nippon Beet Sugar Manufacturing Co., Ltd.; containing 98% raffinose)
Lactulose: Milk Oligosaccharide MLC-97 (produced by Morinaga Milk Industry Co., Ltd.; containing 97% lactulose)
Xylooligosaccharide: Xylo-Oligo 95P (produced by Suntory Wellness Limited; containing 95% xylooligosaccharide)
Agarooligosaccharide: Takara Aga-oligosaccharide® (produced by Takara Bio Inc.; containing 85% agarooligosaccharide)

### <Evaluation procedure>

The quercetin glycoside and each of the different oligosaccharides were mixed at each of the weight ratios shown in Table 1 to thereby prepare different mixed powders. The weight ratios shown in this table are based on the weights of the respective compounds of interest *per se,* which were calculated from the purities of the materials used. Seven panelists orally ingested 1 g each of the prepared mixed powders with water and evaluated the mixed powders for the intensity of the bitterness derived from the quercetin glycoside according to the following criteria.

With the bitterness intensity of a sample consisting of the quercetin glycoside alone being taken as 5 points, the panelists rated the samples on a scale of 0 to 5 points.
0 point: "No bitterness is sensed"
5 points: "Bitterness is sensed at the same intensity as that of the quercetin glycoside-alone sample."
The scores for the samples were compiled by averaging the ratings given by the seven panelists. The results are shown in Table 1.

**[Table 1]**

| Sample No. | 1 | 2 | 3 | 4 | 5 | 6 | 7 | 8 |
|---|---|---|---|---|---|---|---|---|
| Quercetin glycoside : Oligosaccharide | 1 : 0 | 1 : 5 | 1 : 5 | 1 : 5 | 1 : 5 | 1 : 5 | 1 : 5 | 1 : 5 |
| (1) Quercetin glycoside | 1 | 1 | 1 | 1 | 1 | 1 | 1 | 1 |
| (2) Fructooligosaccharide | | 5 | | | | | | |
| (3) Maltooligosaccharide | | | 5 | | | | | |
| (4) Paratinose | | | | 5 | | | | |
| (5) Raffinose | | | | | 5 | | | |
| (6) Lactulose | | | | | | 5 | | |
| (7) Xylooligosaccharide | | | | | | | 5 | |
| (8) Agarooligosaccharide | | | | | | | | 5 |
| Sensory evaluation score | 5.0 | 1.7 | 3.4 | 3.1 | 4.4 | 3.1 | 2.7 | 4.3 |

As is evident from the results shown in Table 1, the fructooligosaccharide is significantly high in the masking effect on the bitterness of the quercetin glycoside as compared to the other oligosaccharides.

### Example 2

Investigation was made to identify a relative ratio of the fructooligosaccharide that can advantageously mask the bitterness of the quercetin glycoside in a quercetin glycoside-containing composition. To be specific, evaluation was conducted on samples containing the fructooligosaccharide at varied ratios with respect to the quercetin glycoside, with the raffinose-containing sample which showed little masking effect in Example 1 being used as a control. The materials and evaluation procedure used were the same as in Example 1. The results are shown in Table 2.

**[Table 2]**

| Sample No. | 1 | 2 | 3 | 4 | 5 | 6 | 7 | 8 | 9 | 10 | 11 |
|---|---|---|---|---|---|---|---|---|---|---|---|
| Quercetin glycoside: Oligosaccharide | 1 : 0 | 1 : 0.02 | 1 : 0.04 | 1 : 0.1 | 1 : 0.2 | 1 : 1 | 1 : 5 | 1 : 10 | 1 : 25 | 1 : 50 | 1 : 5 |
| (1) Quercetin glycoside | 1 | 50 | 25 | 10 | 5 | 1 | 1 | 1 | 1 | 1 | 1 |
| (2) Fructooligosaccharide | | 1 | 1 | 1 | 1 | 1 | 5 | 10 | 25 | 50 | |
| (3) Raffinose | | | | | | | | | | | 5 |
| Sensory evaluation score | 5.0 | 4.9 | 4.6 | 3.9 | 3.6 | 3.1 | 1.9 | 1.3 | 1.1 | 1.0 | 4.4 |

As is evident from the results shown in Table 2, it is found that the samples comprising the fructooligosaccharide at ratios of 0.1 or greater with respect to the quercetin glycoside, which is taken as 1, can effectively mask the bitterness of the quercetin glycoside, as compared to the control (Sample No. 11).

### Example 3

Young barley leaf powder, *Ashitaba* powder, and green tea leaf powder were provided as dietary fiber-containing ingredients, and these ingredients were mixed with a quercetin glycoside ("Sanmelin® Powder FA" produced by San-Ei Gen F. F. I., Inc.) and maltitol in the relative amounts shown in Table 3. The resulting mixed powders were each charged into a fluidized bed granulator (FD-LAB-1 produced by Powrex Corporation), and mixing was continued until the product temperature reached 40°C. Next, spray liquids were prepared by dissolving a xylooligosaccharide syrup ("Xylo-Oligo 70" produced by Suntory; solids content: 75%; xylooligosaccharide content in solids: 70%) or a fructooligosaccharide syrup ("Meioligo® G" produced by Meiji Food Materia Co., Ltd.; solids content: 75%; fructooligosaccharide content in solids: 55%) in the relative amounts shown in Table 3. Then, test samples were each prepared such that the total solids content derived from each of the mixed powders and the spray liquids amounted to about 300 g. It should be noted that the weights shown in this table are the weights of the respective compounds of interest *per se,* which were calculated from the purities of the materials used. The granulation conditions are as follows: the granulation was continued for 5 to 15 minutes while each of the spray liquids was sprayed at rates of 10 to 30 mL/min. (product temperature: 35°C to 40°C; charge air temperature: 65°C). After dried for 10 to 20 minutes in the granulator, the granulated products were removed from the granulator and sieved through a No. 20 sieve (with an aperture of 840 µm) to obtain granulates.

The samples were evaluated for the proportion of fine powder as an index of granulability. More specifically, those samples in which the proportion of fine powder passing through a No. 200 sieve (with an aperture of 75 µm) was not greater than 50% by weight based on the total weight were rated as satisfactory in terms of granulability. This criterion was established taking into account the ease of handling at the time of dividedly packing the resulting granules (in a pouch).

The results are shown in Table 3. The compositions comprising young barley leaf powder, *Ashitaba* powder, green tea leaf powder, etc. as well as the xylooligosaccharide or the fructooligosaccharide were evaluated for granulability -- those in which the proportion of fine powder of 75 µm or less was less than 50% were rated as "O", and those in which that proportion was 50% or greater were rated as "X ". The results revealed that the inventive compositions prepared with the fructooligosaccharide can be granulated as satisfactorily as the composition prepared with the xylooligosaccharide.

Further, when these compositions were suspended in water and consumed, they were found to have good flavor of dietary fiber-containing ingredients.

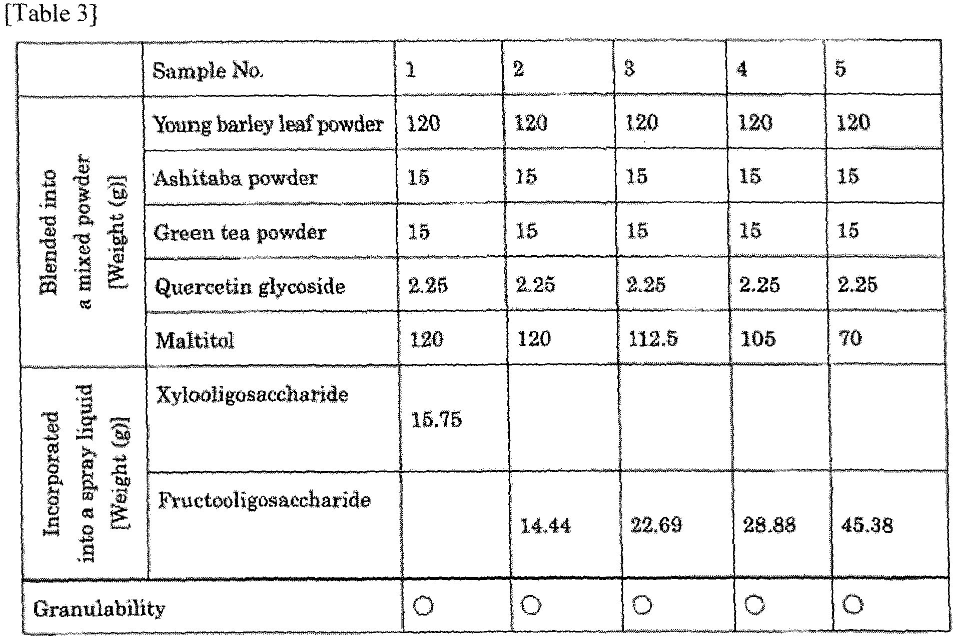

## Claims

1. An oral composition comprising a quercetin glycoside and a fructooligosaccharide, wherein a weight ratio of the fructooligosaccharide to the quercetin glycoside is 0.1 or greater.

2. The oral composition according to claim 1, further comprising a dietary fiber-containing ingredient.

3. The oral composition according to claim 2, wherein the dietary fiber-containing ingredient comprises powder of a green plant.

4. The oral composition according to claim 2 or 3, wherein the dietary fiber-containing ingredient comprises one or more of *Ashitaba (Angelica keiskei),* young cereal leaf, green tea plant, kale, young sweet potato leaf, broccoli, *Corchorus olitorius,* young sesame leaf, mulberry leaf, *Peucedanum japonicum,* and mugwort.

5. The oral composition according to any one of claims 1 to 4, wherein the oral composition has attached thereto a labeling of a function based on: prevention or amelioration of symptoms such as arteriosclerosis, anemia, gastritis, gastric ulcer, and constipation; improvement in liver functions; inhibition of toxic substances adsorption; improvement in intestinal environment; dieting effect; inhibition of cholesterol absorption; prevention of a rapid elevation of postparandial blood glucose level; activation of superoxide dismutase; intestinal regulation activity including bifidobacterium growth activity; or colorectal cancer-preventing, mineral absorption-promoting, or antioxidative activity.

6. The oral composition according to any one of claims 1 to 5, wherein the oral composition is in the form of a granule or a tablet.

7. A method for suppressing bitterness of a quercetin glycoside-containing oral composition, comprising mixing the quercetin glycoside and a fructooligosaccharide at a weight ratio of the fructooligosaccharide to the quercetin glycoside being 0.1 or greater.
